(19) Europäisches Patentamt / European Patent Office / Office européen des brevets

(11) **EP 3 911 432 B1**

(12) **EUROPEAN PATENT SPECIFICATION**

(45) Date of publication and mention of the grant of the patent:
**13.08.2025 Bulletin 2025/33**

(21) Application number: **20700516.6**

(22) Date of filing: **17.01.2020**

(51) International Patent Classification (IPC):
**B01D 61/24** $^{(2006.01)}$    **A61M 1/16** $^{(2006.01)}$
**B01D 63/02** $^{(2006.01)}$    **B01D 65/02** $^{(2006.01)}$
**B01D 67/00** $^{(2006.01)}$    **B01D 69/02** $^{(2006.01)}$
**B01D 71/68** $^{(2006.01)}$

(52) Cooperative Patent Classification (CPC):
**B01D 61/243; A61M 1/168; B01D 61/244;
B01D 63/023; B01D 65/022; B01D 67/00111;
B01D 69/02; B01D 71/68;** B01D 2325/38

(86) International application number:
**PCT/EP2020/051078**

(87) International publication number:
**WO 2020/148411 (23.07.2020 Gazette 2020/30)**

(54) **DIALYZER COMPRISING A FLUORINE-CONTAINING HOLLOW FIBER MEMBRANE**

DIALYSATOR MIT EINER FLUORHALTIGEN HOHLFASERMEMBRAN

DIALYSEUR COMPRENANT UNE MEMBRANE À FIBRES CREUSES CONTENANT DU FLUOR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.01.2019 EP 19152603**

(43) Date of publication of application:
**24.11.2021 Bulletin 2021/47**

(73) Proprietor: **Fresenius Medical Care Deutschland GmbH
61352 Bad Homburg (DE)**

(72) Inventors:
- **KENNEDY, James**
  **Salt Lake City, UT 84124 (US)**
- **SANDER, Roland**
  **66606 St. Wendel (DE)**
- **MEISINGER, Sebastian**
  **66646 Marpingen (DE)**
- **KELLER, Torsten**
  **66606 St. Wendel (DE)**

(74) Representative: **Ricker, Mathias
Wallinger Ricker Schlotter Tostmann
Patent- und Rechtsanwälte Partnerschaft mbB
Zweibrückenstraße 5-7
80331 München (DE)**

(56) References cited:
EP-A1- 2 295 132    WO-A1-2018/029133
DE-A1- 102016 224 627    US-A- 5 954 966

- KHULBE K. C. ET AL: "Characterization of surface-modified hollow fiber polyethersulfone membranes prepared at different air gaps", JOURNAL OF APPLIED POLYMER SCIENCE, vol. 104, no. 2, 15 April 2007 (2007-04-15), US, pages 710 - 721, XP093100314, ISSN: 0021-8995, DOI: 10.1002/app.24853
- J. Y.HO ET AL: "The effect of fluorinated surface modifying macromolecules on the surface morphology of polyethersulfone membranes", JOURNAL OF BIOMATERIALS SCIENCE. POLYMER EDITION., vol. 11, no. 10, 2 January 2000 (2000-01-02), NL, pages 1085 - 1104, XP055604843, ISSN: 0920-5063, DOI: 10.1163/156856200743599

- **VU ANH PHAM ET AL: "Application of surface modifying macromolecules in polyethersulfone membranes: Influence on PES surface chemistry and physical properties", JOURNAL OF APPLIED POLYMER SCIENCE, vol. 73, no. 8, 22 August 1999 (1999-08-22), US, pages 1363 - 1378, XP055604778, ISSN: 0021-8995, DOI: 10.1002/(SICI)1097-4628(19990822)73:8<1363::AID-APP3>3.0.CO;2-P**

**Description**

## FIELD OF THE INVENTION

The invention relates to a dialyzer comprising hollow fiber membranes for the treatment of blood. More specifically, the invention relates to a dialyzer with improved antithrombogenic properties, and methods of making dialyzers comprising sterilizing said dialyzers.

## BACKGROUND OF THE INVENTION

Various blood purifying methods have been proposed for a treatment of a patient suffering from renal failure, in which blood to be purified is taken out from the living body of the patient, is purified and then the purified blood is returned into the body. The blood purification methods utilizing extracorporeal circulation are implemented using a hemodialyzer.

The dialyzer purifies the blood from waste solutes and uremic toxins (e.g., urea, potassium, creatinine, uric acid, and "middle molecule proteins"). Commonly, all modern dialyzers in use are of the hollow-fiber variety. Herein, a cylindrical bundle of hollow fibers, whose walls are composed of semi-permeable membranes, is anchored at each end into a potting compound. This assembly is then put into a clear plastic cylindrical housing with four openings. One opening or blood port at each end of the cylinder communicates with each end of the bundle of hollow fibers. This forms the *"blood compartment"* of the dialyzer. Two other ports are cut into the side of the cylinder. These communicate with the space around the hollow fibers, the *"dialysate compartment."* The corresponding membranes therefor exhibit a "blood side" and a dialysate side". Blood is pumped via the blood ports through this bundle of very thin capillary-like tubes, and the dialysate is pumped through the space surrounding the fibers. Pressure gradients are applied when necessary to move fluid from the blood to the dialysate compartment.

While the blood is being transported from and to the body and cleaned in the dialyzer, an anticoagulant, such as heparin, is frequently added to prevent thrombosis or clotting of the filter. While advantageous, the use of heparin in some patients can cause allergic reactions and bleeding, and can further be contraindicated for use in patients taking certain medications. In an acute therapy for poly-traumatized patients, the use of heparin during dialysis is very often contra-indicated due to excess bleeding.

EP 2 295 132 A1 proposes antithrombogenic extracorporeal blood circuits and components thereof, such as hollow fiber membranes, blood tubing, and filters, and their use in hemofiltration, hemodialysis, hemodiafiltration, hemoconcentration, blood oxygenation, and related uses, wherein the use of an anticoagulant may be avoided. The hollow fiber membrane defined in this document comprises a fluorine-containing macromolecule. To achieve a sufficient effect for the reduction of the thrombogenic effect, a fairly high amount of such fluorine-containing macromolecule has to be used.

Khulbe et al. (J. Appl. Polym. Sci., vol. 104, no. 2, pages 710-721) discloses further hollow fiber membranes comprising fluorine-containing macromolecules and DE102016224627 discloses the steam-sterilization of dialyzers.

## OBJECTS OF THE INVENTION

Due to the importance of hemodialysis and the steady increasing number of patients suffering from renal failure including the risk of thrombogenicity, there is an ongoing need for providing cost-optimized dialysis filters comprising hollow fiber membranes that have a thrombogenicity as low as possible.

## SUMMARY OF THE INVENTION

The inventors of the present invention discovered that sterilization of dialysis filters comprising hollow fiber membranes has an impact on hemocompatibility and in particular on thromobogenicity. This particularly applies when said hollow fiber membrane comprises a fluorine-containing macromolecule.

More specifically, the inventors discovered that the amount of a surface modifying macromolecule (SMM) present in the spinning mass and in the membrane material can be reduced when using the process of steam sterilizing for the production of the membranes and dialyzers instead of the commonly used method of sterilizing with high energy rays like γ- rays or e-beam radiation.

In the following, all terms in quotation marks as used in this disclosure are defined in the meaning of the invention.

## BRIEF DISCUSSION OF THE FIGURES

In the figures show

Fig. 1      the influence of the concentration of SMM1 (a fluorine-containing macromolecule commercially available from Interface Biologics, Toronto, Ont., Canada) in a spinning mass from which hollow fiber membranes are prepared on contact angle θ of the membrane when the membrane is subjected to steam and e-beam sterilization;

Fig. 2      the influence of the concentration of SMM1 in a spinning mass from which hollow fiber membranes are prepared on surface potential ζ of the membrane when the membrane is subjected to steam and e-beam sterilization;

Fig. 3      the influence of the concentration of SMM1 in a spinning mass from which hollow fiber membranes are prepared on Na clearance of the membrane when the membrane is subjected to steam and e-beam sterilization;

Fig. 4      the influence of the concentration of SMM1 in a spinning mass from which hollow fiber membranes are prepared on vitamin B12 clearance of the membrane when the membrane is subjected to steam and e-beam sterilization;

Fig. 5a      the influence of the concentration of SMM1 in a spinning mass from which hollow fiber membranes are prepared on hemocompatibility in terms of complement activation of the membrane when the membrane is subjected to steam and e-beam sterilization; the values are given in relation to an e-beam sterilized filter;

Fig. 5b      the influence of the concentration of SMM1 in a spinning mass from which hollow fiber membranes are prepared on hemocompatibility in terms of complement activation of the membrane when the membrane is subjected to steam and e-beam sterilization; the values are given in relation to a FX60 filter (commercially available from Fresenius Medical Care, Bad Homburg, Germany);

Fig. 6a      the influence of the concentration of SMM1 in a spinning mass from which hollow fiber membranes are prepared on hemocompatibility in terms of platelet loss of the membrane when the membrane is subjected to steam and e-beam sterilization; the values are given for the steam-sterilized filter in comparison to an e-beam-sterilized filter;

Fig. 6b      the influence of the concentration of SMM1 in a spinning mass from which hollow fiber membranes are prepared on hemocompatibility in terms of platelet loss of the membrane when the membrane is subjected to steam and e-beam sterilization; the values are given for the steam-sterilized filter and an e-beam-sterilized filter in relation to a FX60 filter;

Fig. 7      the influence of the concentration of SMM1 in a spinning mass from which hollow fiber membranes are prepared on hemocompatibility in terms of TAT of the membrane when the membrane is subjected to steam in comparison to an e-beam sterilized filter;

Fig. 8a,b      Apparatus for the determination of the zeta potential ζ of a hollow fiber membrane;

Fig. 9      Apparatus for a blood sample to be brought into contact with a dialyzer/membrane;

Fig.10-13      Apparatus and sterilization procedure for the sterilization and conditioning steps of sample dialyzers.

## DETAILED DESCRIPTION OF THE INVENTION

According to a *first aspect*, the invention relates to a method for manufacturing a dialysis filter comprising fluorine-containing hollow fiber membranes, the fluorine-containing hollow-fiber membranes being made by a method comprising at least steps (A) to (C):

(A) preparing a spinning solution comprising an aprotic solvent, a hydrophobic base polymer, a hydrophilic polymer, and a fluorine-comprising surface modifying macromolecule in a concentration from 0.1 to less than 1.12 % w/w:
(B) extruding said spinning solution from an outer annular orifice through a tube-in-orifice spinneret into an aqueous solution, and
(C) isolating the formed hollow fiber membrane,

characterized in that the manufacturing of the dialysis filter comprises a steam sterilization procedure.

The concentration of the fluorine-containing surface modifying molecule in the spinning solution used in step A is from 0.1 to less than 1.12 % % w/w and more preferably from 0.5% w/w or more to less than 1 % w/w. The inventors found that the amount of the fluorine-containing polymer could be significantly reduced when the membranes were steam-sterilized in step (D) without reducing the good properties as far as thrombogenicity is concerned. Therefore a cost-optimized membrane could be achieved with the desired properties.

The inventors surprisingly discovered that for steam sterilization the concentration of the fluorine-containing surface modifying polymer can be lower compared to other sterilization methods such as sterilization by means of electromagnetic radiation such as electron-beam sterilization or sterilization by means of γ-radiation. In some methods, the solubility of the fluorine-containing polymer within the spinning mass is too low to achieve the then necessary concentration of dissolved fluorine-containing surface modifying polymer and thus the desired low thrombogenicity. Accordingly, in one embodiment, the thrombogenicity of the fluorine-containing hollow fiber membrane used in step (D) is reduced compared to a fluorine-containing hollow fiber membrane, which has been subjected to electron-beam sterilization. When treating a patient with a dialysis filter according to the invention, the amount of heparin that has to be injected before the blood enters the dialyzer can either be reduced or avoided in total. The need of use of heparin or the amount of use of heparin depends on the coagulation status of the treated patient. Some patients develop heparin-induced thrombocytopenia (HIT), which makes the reduction or avoidance of heparin even more desirable.

The method of manufacturing dialysis filters according to the invention are easy and cost-effective to produce.

The term *"dialysis filter"* encompasses a filter housing comprising hollow fiber membranes in the form of a hollow fiber membrane bundle, the dialysis filter being configured for use in a dialysis machine which can be used by patients suffering from impaired kidney function.

The hollow fiber membrane bundle contains thousands (e.g., 3,000 to 30,000) individual hollow fiber fiber membranes. Typically, the fibers are fine and of capillary size which typically ranges from about 150 to about 300 microns internal diameter with a wall thickness in the range of about 20 to about 50 microns.

In general, methods for the manufacturing of dialysis filters are known in the art. Known methods of manufacturing dialysis filters comprise the relevant manufacturing steps

- providing a bundle of hollow fiber membranes,
- providing a housing of a dialysis filter, which is in particular a cylindrical shaped housing,
- placing the hollow fiber membrane bundle within the housing,
- potting the end sections of the hollow fiber membrane bundle within the housing with a curable resin.
- optionally mounting further parts to the dialysis filter housing, like end sectional header caps to obtain the dialysis filter

According to the method of the invention, the resulting fluorine-containing hollow fiber membranes obtained according to steps (a) to (C) are steam-sterilized within the dialysis filter. The term *"steam sterilization procedure"* means a process, which encompasses at least subjecting the dialysis filter comprising the fluorine-containing fiber membrane to steam having a temperature above 100 °C for a period of time of at least 2 minutes.

Preferably, the temperature of the steam is in the range of from 105 °C to 150 °C, and the period of time is in the range of from 2 to 20 minutes.

The term *"steam"* means water vapor or encompasses water vapor. Within the meaning of the present application, steam or water vapor also refers to a form of gaseous water, which is accompanied by so-called visible steam vapor; i.e. mist-like water droplets distributed throughout the air. Thus, also encompassed by the term water vapor as it is used in the present application are other sub-designations of water vapor such as, for example, superheated steam, wet steam, saturated steam, overheated steam and supercritical steam.

The membrane obtained by the method of the invention shows a reduced thrombogenicity compared to commonly used sterilization methods. The term *"reducing thrombogenicity"* means that the hollow fiber membrane obtained has improved antithrombogenic properties compared to the fluorine-containing hollow fiber membrane sterilized by rays.

The term *"antithrombogenic"* refers to any extracorporeal blood circuit, or component thereof (e.g., a hollow fiber membrane, blood tubing, dialysis filter, and/or a potted bundle of hollow fiber membranes) for which the rate at which thrombosis occurs upon exposure to whole blood is greatly reduced so that the amount of required heparin can be reduced or the use of heparin in an external blood circuit like a dialyzer circuit can be avoided.

The term *"improved hemocompatability"* is a generic term that encompasses *"reduced thrombogenicity"* or *"improved antithrombogenic properties"*, but also parameters like reductions in the activation of the complement system of the innate immune system are covered by the term.

The measurement of the *"antithrombogenic"* property is accomplished through the determination of the *"platelet loss"* during an experiment, where a sample of blood is directed through a dialysis filter in an extra-corporal circuit. Platelets or thrombocytes are cells in human blood directed to the coagulation system. During an external blood treatment cycle a loss of these platelets is often observed indicating coagulation activities in the device of the extra-corporal system. The platelets are then deposited within the extra-corporal device and may cause a clogging. The clogging effect is a particular problem in extra-corporal filters like a dialyzer. A reduced *"platelet loss"* is therefore an indicator for an improved *"antithrombotic property"* derived from the cell system of the blood. When such a dialyzer is used in a blood purification therapy, the amount of heparin injected into the blood line before the dialyzer can be reduced. In some cases it may be possible to avoid the use of heparin in total.

An indicator of the *"antithrombotic"* property derived from the proteomic system of the blood is the concentration of the "thrombin-antithrombin III" or "TAT III" complex in the blood that has been directed through the extra-corporal circuit. A high concentration of the TAT III complex is a measure for a high activity of the proteomic coagulation system and therefore a measure for a "reduced antithrombotic" property.

A good indicator for the parameter *"hemocompatibility",* as far as the response of the innate immune system is concerned, is the determination of the status of the complement system. The complement system is a proteomic system that can quickly respond to harmful events to the blood. Such a harmful event can be the contact of the blood to the surfaces of the extra-corporal circuit. Therefore very often the complement system is activated during a blood filtration procedure like a dialysis session. A good measure for the activation of the complement system is the measurement of the concentration of the complement fraction C5a, generated during the complement cascade as a reaction to the harmful event. The fragments C3a and C5a act as anaphylactic toxins and can cause a shock situation during the dialysis treatment. Therefore the reduction of these proteins is desired for a good therapy.

Various processes are known in the art for the production of hollow fiber membranes as e.g. summarized in EP 2 295 132 A1 including

(i) processes wherein a tube-in-tube type orifice is used and the spinning solution is extruded from the outer tube (i.e., from the annular space defined between the inner and outer tubes) and the core solution is ejected from the inner tube;

(ii) by extruding the spinning solution into air, allowing the filament to fall down by gravity, passing the filament through a bath with a non-solvent for coagulation, and washing and drying the filament;

(iii) by using a bath including an upper layer of a non-coagulating solution and a lower layer of a coagulating solution, and extruding the spinning solution directly into the non-coagulating solution and passing the filament through the coagulating solution;

(iv) by using a bath including an upper layer of a coagulating solution and a lower layer of a non-coagulating solution, and extruding the spinning solution directly into the non-coagulating solution and passing the filament through the coagulating solution;

(v) by extruding the spinning solution directly into a non-coagulating solution and passing the filament along the boundary between the coagulating solution and the non-coagulating solution; and

(vi) by extruding the spinning solution from the orifice surrounding a non-coagulating solution and passing the filament through a coagulating solution.

The combination of (i) and ii) is called dry-wet spinning, which is the process widely used in the production of hollow fiber membranes for the treatment of blood during a dialysis therapy. The process can be widely adjusted to control the properties of the membrane.

In such processes, the pore diameter of the hollow fiber membrane is controlled by adjusting the rate and the extent of the coagulation of the extruded spinning solution through the use of a coagulation solution which promotes the coagulation of the spinning solution (a non-solvent for the spinning solution) and a non-coagulation solution which inhibits the coagulation of the spinning solution (a solvent for the spinning solution) either separately or in a mixture. Additionally the coagulation process can be controlled through the adjustment of the temperature of the spinning mass and the core coagulation solution.

A typical spinning solution or spinning mass suitable for making a fluorine-containing hollow fiber membrane according to at least steps (A) to (C) include a hydrophobic base polymer, a hydrophilic pore forming agent (e.g., polyvinylpyrrolidone, polyethyleneglycol, alcohols, polypropylene glycol, or polyethylene glycol), an aprotic solvent for the polymer, and a surface modifying macromolecule comprising fluorine.

Hydrophobic polymers have been widely used as polymeric materials in hollow fiber membranes. In particular, polysulfones (PSU) are synthetic hydrophobic polymers that are predominantly widely used in hollow fiber membranes due to their excellent fiber spinning properties and biocompatibility. The spinning solution used in step (A) of the method of making the fluorine-containing hollow fiber membrane comprises a polysulfone.

The term *"polysulfone (PSU)"* is used as a general term for polymers comprising units of a polymeric aryl sulfone. Thus, the term encompasses a polysulfone made from bisphenol A, a polyethersulfone, made from bisphenol S, polyphenylethersulfone and copolymers made therefrom.

Polysulfone based polymers show in general a good hemocompatibility when used as dialysis filter membranes. It has been found that polysulfones exhibit in particular a good compatibility with fluorine-based surface modifying molecules and polymers yielding membranes with high mechanical strength.

However, pure hydrophobic PSU cannot be used directly for some applications, e.g., dialysis membranes, as this will decrease the wetting characteristics of the membrane in an aqueous environment and negatively affect the wetting properties essential for the clearance of toxins. To address this problem, polyvinylpyrrolidone (PVP) or polyethylene glycol is typically added to the PSU as a hydrophilic polymer rendering at least a part of the membrane surface hydrophilic.

The spinning solution used in step (A) of the method of making the fluorine-containing hollow fiber membrane comprises a polyvinylpyrrolidone. The term *"polyvinylpyrrolidone"* comprises the homopolymer as well as co-polymers like vinylpyrrolidone-vinylacetate-based copolymers. Suitable compounds are known in the art.

The use of hydrophilic polymers and vinylpyrrolidone based polymers in particular is suitable to render the blood side of the membrane more hydrophilic, when the spinning solution comprises fluorine-containing surface modifying polymers. Membranes made by the methods show a good balance of hydrophilicity and hydrophobicity and therefore are well suited as highly hemo-compatible and antithrombotic membranes. This balance is crucially enhanced when the membrane is steam sterilized.

The polyvinylpyrrolidone also supports the pore forming process of the membrane in its entirety and the pore forming process of the selective layer on the blood side in particular.

In one embodiment of the method of manufacturing a dialysis filter according to the invention the fluorine-containing hollow-fiber membranes, are made by the use of an aprotic solvent in step (A) selected from the group consisting of dimethylformamide, dimethylsulfoxide, diemethylacetamide, N-methylpyrrolidone or a mixture of two or more thereof. These solvents are well suited to the production of membranes comprising a fluorine-containing surface modifying macromolecule. The solvent has to be adjusted to provide the desired solubility of the fluorine containing molecule as well as the desired solubility of the hydrophobic base polymer and the hydrophilic polymer.

The spinning solution used in step (A) of the method of making the fluorine-containing hollow fiber membrane and thus the produced fluorine-containing hollow fiber membrane comprises a surface-modifying macromolecule, which is described by formula

$$F_T\text{-}[B\text{-(oligo)}]_n\text{-}B\text{-}F_T$$

wherein

B comprises a urethane;
oligo comprises polypropylene oxide, polyethylene oxide or polytetramethylene oxide;
$F_T$ is a polyfluoroorgano group; and
n is an integer from 1 to 10.

Such a molecule can easily be incorporated into the spinning solution providing the desired effect of improving the anti-thrombotic properties of the membrane. This range of surface modifying molecules yields a balanced property of hydrophilicity and hydrophobicity.

Such suitable surface-modifying macromolecule is preferably made from 1H,1H,2H,2H-perfluorooctanol as $F_T$, hexamethylene diisocyanate as B and propylene oxide as oligo. A respective SMM product is known and commercially available under the trademark Endexo™ from Interface Biologics, Toronto, Ont., Canada. Such polymers are easily obtainable and provide the desired properties for the corresponding membranes. This molecule shows particularly good properties in the desired low concentration in the spinning mass of less than 1.12 % *w/w.* The molecule provides a sufficient solubility in the desired concentration within the solvent mixture necessary for the production of the membrane maintaining the desired filtering characteristics as far as the molecular weight of the uremic toxins is concerned. This is particularly the case when dimethylacetamide is used as an aprotic solvent, Bisphenol-A-Polysulfone or Bisphenol-S-Polysulfone is used as the hydrophobic base polymer and polyvinylpyrrolidone as the hydrophilic polymer in concentrations suitable to form a well performing dialysis membrane.

In a further embodiment of the method of manufacturing a dialysis filter comprising the fluorine-containing hollow fiber membranes, after the method of making the hollow fiber membranes according to steps (A) to (C) at least one of the contact angle of water on the surface of the hollow fiber membrane $\Theta$, the zeta potential $\zeta$, the platelet loss and/or the TAT III concentration is determined. The determination of these membrane properties can ensure that the reduced thrombogenic behavior is maintained during the manufacturing of the dialysis filter. The determination of one of these properties is easy to perform and highly correlated to the desired anti-coagulation properties of the membrane.

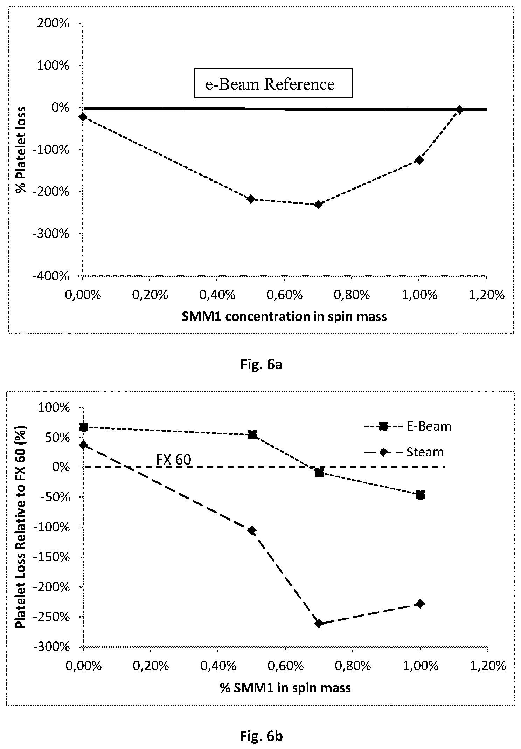

In a further embodiment of the method of manufacturing a dialysis filter comprising the fluorine-containing hollow fiber membranes, the manufacturing of the dialysis filter is characterized in that during the steam sterilization procedure a trans-membrane pressure difference of the water vapor is applied across the membrane walls of the hollow fiber membranes, so that water vapor is conducted into the interior of the fibers and is permeated through the membrane wall to the exterior of the fiber. Such a method of steam sterilizing is described in detail in DE 10 2016 224 627 A1, in particular in Example 1 with regard to Fig. 6 to 9. Applying such a trans-membrane pressure ensures excellent anti-thrombotic properties of the membrane comprising the fluorine-containing surface modifying molecule within the membrane. Obviously some of the lower molecular weight substances are removed through the pores of the membrane from the inside to the outside yielding good balanced hydrophobic-hydrophilic properties of the membrane and therefore an enhanced hemocompatibility.

According to a **second aspect,** the invention relates to a dialysis filter comprising fluorine-containing hollow fiber membranes comprising a hydrophobic base polymer, a hydrophilic polymer and a fluorine-comprising surface modifying macromolecule as defined in the **first aspect,** wherein the concentration of the fluorine-containing surface modifying macromolecule is from 1.5 to less than 3.6% w/w., preferably 3.2% w/w or less, more preferably from 1.5 % w/w. or more to 3.2% w/w. or less, more preferably from 1.5 % w/w. or more to 2.0% w/w. or less, based on the total weight of the fluorine-containing hollow fiber membrane, respectively. The inventors found that despite the low concentration of the surface modifying macromolecule the blood contact side of the membrane is rendered anti-thrombogenic, so that coagulation effects within the inner surface of the membrane are reduced. During the treatment of a patient the amount of administered heparin can be reduced or the treatment with heparin can be avoided in total. The dialysis filters according to the invention are easy and cost-effective to produce.

The hydrophobic base polymer comprises a polysulfone polymer. Polysulfone polymers are widely used as membrane forming polymers for the use in dialysis therapies. The inventors found that the compatibility between polysulfone polymers, in particular bisphenol-A and bisphenol-S-based polysulfones, is enhanced when using a fluorine-containing surface modifying macromolecule. A good balance of the overall properties like good clearance, a good removal of middle molecule uremic toxins and enhanced anti-thrombogenic properties can easily be achieved.

The hydrophilic polymer comprises a polyvinylpyrrolidone. The use of polyvinylpyrrolidone as a hydrophilic polymer is widely known in the art. The polyvinylpyrrolidone according to the art modifies the surface of the blood contact side of a dialysis membrane more hydrophilic. When using a fluorine-containing surface-modifying macromolecule the polyvinylpyrrolidone is not able to render the surface more hydrophilic but provides a good pore size distribution, so that the desired properties like the removal of uremic middle molecule toxins and the clearance of low molecular weight molecules can be achieved.

The surface-modifying macromolecule is described by formula

$$F_T\text{-}[B\text{-}(oligo)]_n\text{-}B\text{-}F_T$$

wherein

B comprises a urethane;
oligo comprises polypropylene oxide, polyethylene oxide or polytetramethylene oxide; $F_T$ is a polyfluoroorgano group; and
n is an integer from 1 to 10.

Such a molecule can easily be incorporated into the spinning solution providing the desired effect of improving the anti-thrombotic properties of the membrane. This range of surface modifying molecules yield a balanced property of hydrophilicity and hydrophobicity.

In another embodiment, for the fluorine-containing hollow fiber membrane, $F_T$ is represented by 1H,1H,2H,2H-perfluorooctanol, B is represented by a hexamethylene diisocyanate based urethane and oligo is represented by polypropylene oxide. A respective SMM product is known and commercially available under the trademark Endexo™ from Interface Biologics, Toronto, Ont., Canada. Such polymers are easily obtainable and provide the desired properties for the corresponding membranes. This molecule shows particularly good properties in the desired low concentration in the spinning mass of less than 1.12 % w/w. The molecule provides a sufficient solubility in the desired concentration within the solvent mixture necessary for the production of the membrane maintaining the desired filtering characteristics as far as the molecular weight of the uremic toxins is concerned. This is particularly the case when dimethylacetamide is used as an aprotic solvent, Bisphenol-A-Polysulfone or Bisphenol-S-Polysulfone is used as the hydrophobic base polymer and polyvinylpyrrolidone as the hydrophilic polymer in concentrations suitable to form a well performing dialysis membrane.

In one embodiment, the fluorine-containing hollow fiber membrane exhibits a contact angle $\theta$ of water on the surface of the hollow fiber membrane of more than 70°, preferably more than 72°; or a zeta potential $\zeta$ in the range of more than - 5.0 mV, preferably in the range of more than -4 mV; or a contact angle $\theta$ of water on the surface of the hollow fiber membrane of more than 70°, preferably more than 72°, and a zeta potential $\zeta$ of more than - 5.0 mV, preferably of more than -4 mV;

the determination method of the contact angle $\theta$ and the determination method of the zeta potential $\zeta$ being specified in the section EXAMPLES.

It has been found that a reduced thrombogenicity can additionally be estimated by measuring the contact angle $\theta$ of a liquid on the hollow fiber membrane or can be correlated to such measurement.

The term *"contact angle $\theta$"* in general means the angle which is formed between a solid surface in contact with a liquid, in particular water. The measurement method for the contact angle $\theta$ is described in detail below in the section EXAMPLES.

It has been found, that a desired contact angle, measured by the method disclosed in the section EXAMPLES, is more than 70°, preferably more than 72°, with water as the test liquid. In contrast to these embodiments membranes in the prior art without a fluorine-containing surface modifying molecule are treated to become more hydrophilic with contact angles of less than 65° and preferably less than 55°. The blood contact surface of the membrane according to the invention is therefore rendered more hydrophobic. This hydrophobic effect reduces the interaction of blood components with the surface of the blood contact side of the membrane. For such a hydrophobic membrane a reduced platelet loss and a reduced TAT III generation is achieved rendering the surface less thrombogenic. When treating a patient with such a dialysis filter, the amount of heparin that has to be injected before the blood enters the dialyzer can either be reduced or avoided in total.

It has further been found that in one embodiment of the present invention, a reduced rate of thrombosis, i.e. reduced thrombogenicity, can be estimated or forecast by measuring the zeta potential $\zeta$ or correlates to the zeta potential $\zeta$.

The term *"zeta potential $\zeta$"* in general is the potential difference between test fluid directed along the surface to be examined at a defined velocity. The potential difference between the inlet and the outlet of the test setup is measured. The details are described in the section EXAMPLES.

It has been found that a desired zeta potential is higher than -5 mV and less than 0 mV, preferably higher than -4 mV and less than 0 mV. The surface modifying molecule renders the surface of the blood contact side of the membrane more hydrophobic and more neutral at the same time. Higher in this case means more neutral, but positive zeta potentials should be avoided in total, because this can cause undesired adsorption of blood proteins and cells that can lead to severe negative effects for the patient. For such a "neutralized" membrane a reduced platelet loss and a reduced TAT III generation is achieved rendering the surface less thrombogenic. When treating a patient with a dialysis filter according to the invention, the amount of heparin that has to be injected before the blood enters the dialyzer can either be reduced or avoided in total.

In another embodiment the fluorine-containing hollow fiber membrane has an amount of the surface modifying macromolecule of more than 1.5% w/w. and less than 3.2% w/w., based on the total weight of the fluorine-containing membrane, and a zeta potential of more than -5 mV, preferably more than -4 mV, and a contact angle of more than 70°, preferably more than 72°.

In another embodiment the fluorine containing hollow fiber membrane has an amount of the surface modifying macromolecule of more than 1.5% w/w. and less than 2.0% w/w. and a zeta potential of more than -5 mV, preferably more than -4 mV, and a contact angle of more than 70°, preferably more than 72°.

In another embodiment the fluorine-containing hollow fiber membrane has an amount of the surface modifying macromolecule of more than 1% w/w. and less than 2.0% w/w. and a zeta potential of more than -5 mV, preferably more than -4 mV, and a contact angle of more than 70°, preferably more than 72°.

In another embodiment the fluorine-containing hollow fiber membrane has an amount of the surface modifying macromolecule of more than 1% w/w. and less than 3.2 % w/w. and a zeta potential of more than -5 mV, preferably more than -4 mV, and a contact angle of more than 70°, preferably more than 72°.

Methods of forming a dialysis filter are known in the art, and are e.g. described in EP 2 295 132 A1. A fiber bundle is inserted into a tubular housing and the ends of the fibers are closed. Then a potting mass is inserted into the housing in order to separate the dialysate and the blood compartment. After the potting process the fiber ends are opened again and the housing is closed with end caps. Then the dialyzer is ready to be sterilized.

In a further embodiment, the invention relates to a dialysis filter comprising steam-sterilized fluorine-containing hollow fiber membranes, providing for a reduction of platelet loss of at least 10 %, preferably more than 100%, when subjected to blood compared to a fluorine-containing hollow fiber membrane which has the same composition and is otherwise identical but which has been subjected to a radiation sterilization process, the determination of the platelet loss being specified in the description.

Reference numerals:

| 1 | hollow fiber membrane filter |
| 2, 2a | fluid ports |
| 3, 3a | fluid ports |
| 4, 4a | Ag/AgCl electrode |
| 5, 5a | port for pressure gauge |
| 6 | voltmeter |
| 7 | manometer |
| 8 | tank |

| 51 | apparatus for determining platelet loss |
| 52 | dialyzer |
| 53 | system of hoses |
| 54 | hose pump |
| 55 | blood sample collection point |
| 56 | reservoir for blood |
| 57 | pressure sensor |
| 58 | blood inlet |
| 59 | pressure sensor |
| 60 | blood outlet |
| 61, 62 | ports |

| 100, 200, 300, 400 | apparatus |
| 101, 201, 301, 401 | connection |
| 101a, 201a, 301a, 401a | connector |
| 102, 202, 302, 402 | connection |
| 103, 203, 303, 403 | connection |
| 104, 204, 304, 404 | connection |
| 105, 205, 305, 405 | valve |
| 106, 206, 306, 406 | valve |
| 107, 207, 307, 407 | valve |
| 108, 208, 308, 408 | valve |
| 109, 209, 309, 409 | line |
| 110, 210, 310, 410 | line |
| 111, 211, 311, 411 | line |
| 112, 212, 312, 412 | line |
| 113, 213, 313, 413 | hollow fiber membrane filter, i.e. dialysis filter |
| 114, 214, 314, 414 | fluid port |
| 115, 215, 315, 415 | fluid port |
| 116, 216, 316, 416 | bubble detector |
| 117, 217, 317, 417 | fluid port |
| 118, 218, 318, 418 | fluid port |
| 119, 219, 319, 419 | first chamber |
| 120, 220, 320, 420 | second chamber |
| 121, 221, 321, 421 | hollow fiber membrane |

### EXAMPLES

### General method of making a fluorine-containing hollow fiber membrane

A fluorine containing hollow fiber membrane was prepared using a spinning mass (= spinning solution) of 16 % by weight polysulfone (P3500 from Solvay), 4.00 % by weight polyvinylpyrrolidone (K82-86 from Ashland) with 0; 0,5; 0,7; 1.0; 1.12 % by weight of a fluorine-containing macromolecule, based on the total weight of the spinning mass. The polymer mixture is filled up to 100% with dimethylacetamide (DMAC). This fluorine-containing macromolecule was prepared according to EP 2 295 132 A1 using 1H, 1H,2H,2H-perfluorooctanol, hexamethylene diisocyanate and polypropylene oxide as starting materials. The macromolecule is called SMM1 and is obtained from Interface Biologics.

The spinning mass was heated to 60°C and degassed so as to process it into a homogeneous spinning mass. The spinning mass is extruded through an annular spinneret with a centrally controlled precipitant consisting of 35% DMAC and 65% water as the core liquid. The temperature of the annular spinneret is 70°C. The extruded strand is guided through a precipitation gap, the atmosphere of which is set at a relative humidity of 100%. The height of the precipitation gap is 200 mm; a precipitation gap dwell time of 0.4 sec. is set. The strand is introduced into the precipitating bath consisting of water which is temperature-controlled to 80°C and solidified into a hollow fiber membrane. The hollow fiber membrane is then routed through rinsing baths which are temperature-controlled to a temperature of 75°C to 90°C. The hollow fiber membrane thereafter undergoes a drying process between 100°C and 150°C. The hollow fiber membrane obtained is then crimped using a wavelength of 6.4 mm and thereafter taken up on a coiler and formed into a fiber bundle. Each fiber bundle consisted of 10,752 fibers and the final surface of the filters was 1.37 m$^2$. Those bundles were potted as described in the method "zeta potential".

The content of the fluorine-containing macromolecule has been determined by combustion analysis. The results are shown in the table below. The yield of the SMM1 within the spinning process is around 60%.

| % SMM1 in spinning mass | % SMM1 in the fiber |
|---|---|
| 0.5 | 1.5 |
| 0.7 | 2.0 |
| 1.0 | 3.2 |
| 1.12 | 3.6 |

## Steam sterilization

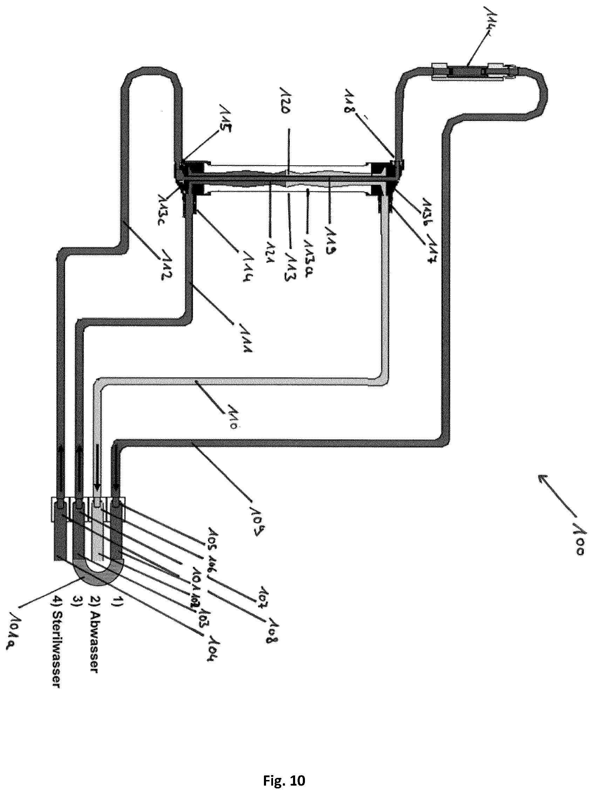

**Fig. 10** schematically depicts a first step of a steam sterilization procedure of a dialysis filter comprising fluorine-containing hollow fiber membranes applied in the manufacturing of the dialysis filter according to the *first aspect* of the invention (*Rinse 1*) using apparatus 100. Schematically a single hollow fiber membrane 121 is shown which shall represent a multitude of hollow fiber membranes formed to a hollow fiber membrane bundle placed inside the housing of the dialysis filter. **Fig. 10** shows a fluid port 118 to a first chamber 119 of a hollow fiber membrane filter 113, which is synonymous for the dialysis filter, encompassing the interior of the hollow fiber membranes, which is fluidly connected to a connection 101 by means of a line 109 having a valve 105. A further fluid port 117 is fluidly connected to connection 102 via line 110, valve 106, and forms an inlet to a second chamber 120 of the hollow fiber membrane filter 113 encompassing a space between the hollow fiber membranes. A further fluid port 114 is in fluidic connection via line 111 with valve 107 and connection 103 and forms an inlet to a second chamber 120 of the hollow fiber membrane filter. Fluid port 115 is in fluidic connection via line 112 with valve 108 and connection 104. Connection 101 and 103 are further in fluidic connection via connector 101a.

In the rinsing procedure, a rinsing liquid (water) is conveyed to the hollow fiber membrane filter 113 through line 112 via connection 104 in the first step as depicted. Preferably, the rinsing liquid is temperature-controlled sterile water, whereby temperatures of 50 to 98°C are maintained. Valve 108 is thereby switched to flow-through. The rinsing liquid flows into the first chamber 119 of the hollow fiber membrane filter via the second fluid port 115 and exits said first chamber via the first fluid port 118. The arrangement enables rinsing the interior of all the hollow fiber membranes of a hollow fiber membrane bundle, potted into a filter housing.

The rinsing liquid further passes through a bubble detector 114, which assumes no function in this rinsing operation, and line 109, and is directed through connection 101 and connector 101a to line 111. The rinsing liquid enters into the second chamber 120 of the filter module 113 via fluid port 114 and flushes the second chamber formed in the space between the hollow fiber membranes. A return flow of the flushing liquid occurs via fluid port 117 and line 110 which is then either discarded or treated so as to again be available for a further rinsing operation.

Sterile water is introduced in connection 104, and waste water is discharged from line 110.

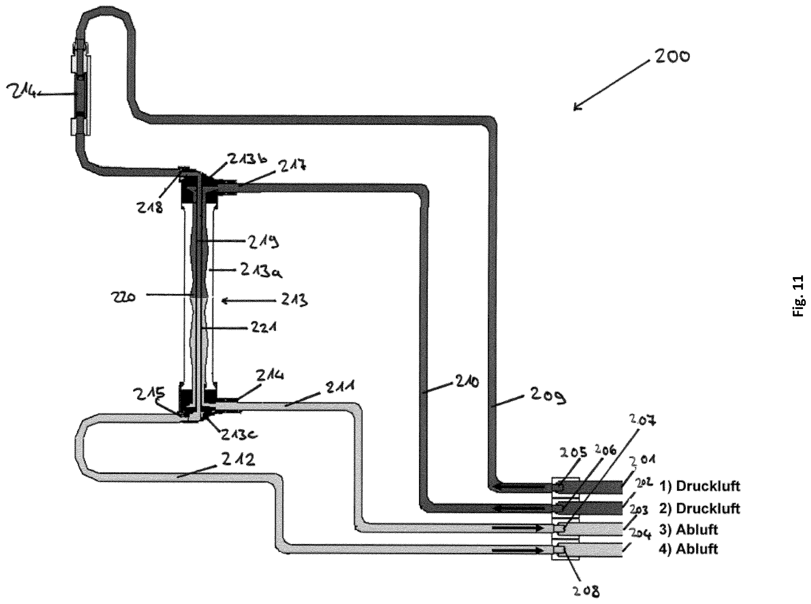

**Fig. 11** schematically depicts a second step of a steam sterilization procedure used in the manufacture of a dialysis filter comprising a fluorine-containing hollow fiber membrane bundle according to the *first aspect* of the invention using apparatus 200. Schematically a single hollow fiber membrane 221 is shown which shall represent a multitude of hollow fiber membranes formed to a hollow fiber membrane bundle placed inside the housing of the dialysis filter. **Fig. 11** is hereby used to illustrate compressed air flushing ("Tempering 1"). A compressed air source feeding sterile air supplies connections 201, 202. The compressed air is conveyed through lines 209 and 201 via the open valves 205 and 206 and to the hollow fiber membrane module 213, which is synonymous for the dialysis filter, by not shown pumping means. The first chamber 219 and the second chamber 220 are initially still filled with water from the preceding rinsing step of the rinsing operation pursuant to Fig. 11. Valves 207 and 208 are open and prepared for a discharge of rinsing liquid. The compressed air is conveyed through the filter module at a pressure of 1.5 to 2 bar (values as examples). Via the respective fluid ports 218, 217, the compressed air thereby further transports residual water out of the first and second chamber of the hollow fiber membrane filter through the respective fluid ports 215 and 214 to the return flow portion of the flow path circuit. Residual water and compressed air are drained off via lines 212, 211. The rinsing process runs for 0,5 to 5 minutes. Since equal pressure prevails in both chambers 219 and 220, there is no flushing across the membrane wall. In consequence, the pores of the membrane wall remain filled with water from the rinsing procedure.

Exhaust air is discharged via connections 203 and 204.

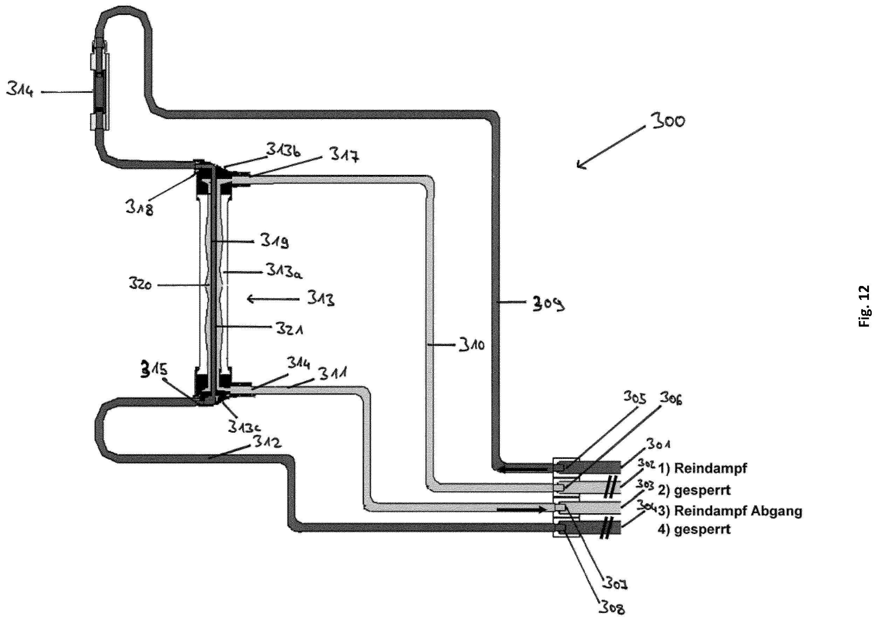

Fig. 12 schematically depicts a third step of a steam sterilization procedure used in the manufacture of a dialysis filter comprising a fluorine-containing hollow fiber membrane bundle according to the *first aspect* of the invention using apparatus 300. Schematically a single hollow fiber membrane 321 is shown which shall represent a multitude of hollow fiber membranes formed to a hollow fiber membrane bundle placed inside the housing of the dialysis filter Fig. 12 depicts the connections 302 and 304 which block the drainage of rinsing fluids by valves 306 and 308 being in a closed valve position. Water vapor is conveyed into the sterilization system via connection 301 and to the filter module 313, which is synonymous for the dialysis filter, via line 309. The water vapor disperses in the first chamber 319 of the hollow fiber membrane filter; drainage via fluid port 315 is not possible since connection 304 is blocked. Water vapor dispersal in line 312 can only ensue by compressing the pressurized pure steam or by diffusion.

Since there is a higher pressure in the first chamber than in the second chamber, a transmembrane passage of pure steam occurs. Residual water remaining in the pores from the rinsing process according to the first step of the rinsing and sterilization operation pursuant to Fig. 12 is evacuated and transported in line 311 through the second chamber 320. Due to connection 302 being closed, line 310 does not thereby serve in conveying fluid. Adjacent hollow fiber membranes are largely separated from one another by the transmembrane flushing procedure. Water vapor is thereby conveyed into the filter module 313 at a pressure of 1.3 to 2 bar (example). The thorough flushing of the pores additionally prevents adhesion of the hollow fibers. This rinsing process can be terminated after a few minutes. In particular, the rinsing procedure is performed for 2 to 5 minutes. Temperatures are kept at 50°C to 98°C, particularly also to thermally condition the filter module for the following sterilization procedure (step "Steam").

Water vapor is discharged via connection 304.

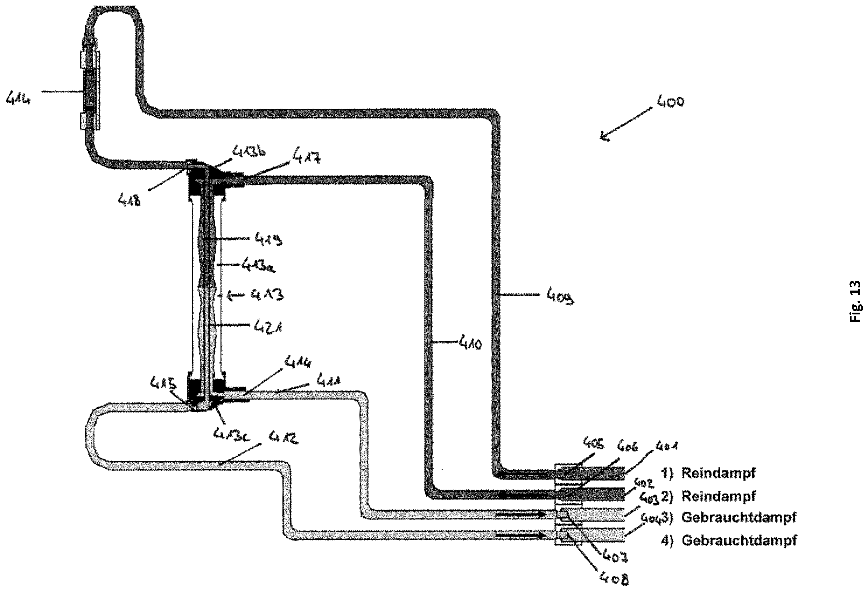

Fig. 13 schematically depicts a fourth step of a steam sterilization procedure as is used in the manufacture of a dialysis filter comprising a fluorine-containing hollow fiber membrane bundle according to *first aspect* of the invention using apparatus 400. Schematically a single hollow fiber membrane 421 is shown which shall represent a multitude of hollow fiber membranes formed to a hollow fiber membrane bundle placed inside the housing of the dialysis filter According to the fourth step, a sterilizing fluid such as, for example, water vapor at a temperature of 124°C and a pressure of 2 bar (as an example) is conveyed into the hollow fiber membrane filter via connections 401 and 402. Waste vapor is discharged via connections 403 and 404. Flow-through is thereby possible through connections 401, 402, 403, 404 by way of the open valves 405 to 408. The pure steam is conveyed to the hollow fiber membrane filter via lines 409 and 410 and the first chamber 419 and the second chamber 420 of the filter module 413, which is synonymous for the dialysis filter flushed. The pure steam is returned via lines 412 and 411 and fluid ports 415 and 414 and is either discarded or treated for reuse. Depending on the sterilization temperature selected, the sterilization procedure can last 5 to 30 minutes. At the preferential temperature of 124°C, sterilization can be considered completed after up to 12 minutes (step "Sterilization"). Further flushing steps can follow in order to bring the hollow fiber membrane filter into a purified and sterile form for use (step "Rinse 1").

For the further quality testing, a "bubble point" test as known from the prior art follows. This test constitutes a pressure hold test in which one side of a membrane is subjected to gas at a higher pressure than the opposite side of the membrane with the fluid flow. The second chamber 120, 220, 320, 420 of the hollow fiber membrane filter shown in Fig. 10 to Fig. 13 is thereby flushed with sterile compressed air, whereby the first chamber remains filled with liquid from the rinsing process. The sterilization system applies a higher pressure in the second chamber than in the first chamber 119, 219, 319, 419. Since the pores are filled with water from the preceding rinsing step, the compressed gas from the first chamber does not breach the second chamber until the pressure applied overcomes the surface tension of the water in the pores. The volume of gas passing into the first chamber can be analyzed in the bubble detectors 114, 214, 314, 414 as shown and the results evaluated accordingly. By correlating the volume of gas bubbles detected to an applied pressure in the second chamber of the filter modules 120 to 420, conclusions can be drawn as to the quality of the membrane material and decisions made as to whether the filter module meets the specification standards (step "Dialysate blow out /Bubble point").

It can subsequently be provided for the first chamber to likewise be flushed with sterile compressed air (step "Tempering 1"). In appropriate cases, a further flushing step with pure steam can ensure the removal of water remaining from preceding rinsing processes (step "steam"). Thereafter, a drying process can occur in which the filter module is flushed with sterile compressed air until a desired degree of dryness is reached (step "Drying").

Data given in the description above act as examples for possible procedures. The following table summarizes the method of steam sterilizing the filters according to the examples of the invention.

| Program Step | Time (Sec) | Media | Description |
|---|---|---|---|
| Rinse 1 | 55 | Water | 90 °C |

(continued)

| Program Step | Time (Sec) | Media | Description |
|---|---|---|---|
| | | | 3,500 mbar<br>2.0 l/min from blood side<br>6.0 l/min from dialysate side<br>Dialysate outlet closed, entire water to the blood outlet |
| Tempering 1 | 55 | Air | 3,000 mbar<br>115.0 °C |
| Steam | 55 | Steam | 1,500 mbar |
| Sterilization | 550 | Steam | 2,400 mbar<br>124.1°C |
| Rinse 1 | 55 | Water | As described before |
| Dialysate blow out / Bubble Point | 55 | Air/Water | Air: 300 mbar<br>Air: 115 °C |
| Tempering 1 | 55 | Air | 3,000 mbar<br>115.0 °C |
| Steam | 55 | Steam | 1,500 mbar |
| Drying | 1100 | Air | 3,000 mbar<br>115.0 °C |

## Electron-beam sterilization (comparative examples)

Electron-beam sterilization was performed using standard conditions, whereby each filter received at least a dose of 26.1 kGy and maximum 43.8 kGy. Prior to the e-beam sterilization the filters were rinsed and conditioned on a commercially available automated conditioning stand using the following settings:

| Program Description | Time (Sec) | Media | Description |
|---|---|---|---|
| Tempering 1 | 110 | Air | 3,000 mbar<br>115.0 °C |
| Conditioning | 205 | Steam | 2,400 mbar<br>124.1°C |
| Rinse 2 | 110 | Water | 80° C<br>3,500 mbar<br>11.0 l/min blood and dialysate<br>Dialysate outlet open |
| Rinse 1 (only blood) | 110 | Water | Like before |
| Dialysate blow out / Bubble Point | 55 | Air/Water | Air: 300 mbar<br>Air: 115°C |
| Tempering 1 | 55 | Air | 3,000 mbar<br>115.0 °C |
| Drying | 1100 | Air | 3,000 mbar<br>115.0 °C |

The steps for the comparative examples were performed according to the steps for the example filters with the exemption of step "Conditioning" and "Rinse 2". The conditioning step is greatly reduced compared to the "Sterilization step" according to the inventive examples, so that no sufficient sterilization takes place. The sterilization is accomplished through the e-beam treatment.

**Test summary**

After samples were prepared, dialyzers from each sterilization group were randomly assigned to testing for contact angle (n=1 dialyzer, 5 fibers), surface (zeta) potential (n=3), clearance (n=3), and hemocompatibility (n=3). The results from each of the tests are shown in the table below:

| SMM1 % | Contact angle $\Theta$ (n=5) | Surface potential $\zeta$ (n=3) | Na-Clearance (n=3) | B12-Clearance (n=3) | Blood test (n=3, E-beam filter with same [SMM1] used as reference) | | |
|---|---|---|---|---|---|---|---|
| | | | | | Platelet Loss (%) | C5a rise/hr (%) | TAT (%) |
| **Steam sterilization** | | | | | | | |
| 1,12 % | 81.00,3 $\pm$ | -3.29 | 260 $\pm$ 2.8 | 164 $\pm$ 1.1 | -5 $\pm$ 9% | -20 $\pm$ 24% | -10 $\pm$ 34% |
| 1,00 % | 80.8 $\pm$ 0,5 | -3.33 | 260 $\pm$ 0.7 | 163 $\pm$ 1.1 | -125 $\pm$ 186% | 16 $\pm$ 11% | -70 115% |
| 0,70 % | 75.8 $\pm$ 0,9 | -3.84 | 269 $\pm$ 2.0 | 172 $\pm$ 1.3 | -231 $\pm$ 151% | -7 $\pm$ 24% | -70 $\pm$ 59% |
| 0,50 % | 72.1 $\pm$ 0,6 | -4.2 | 268 $\pm$ 1.3 | 171 $\pm$ 0.4 | -218 $\pm$ 141% | -37 $\pm$ 77% | -108 $\pm$ 106% |
| 0,00 % | 49.0 $\pm$ 4.9 | -6.09 | 267 $\pm$ 1.9 | 172 $\pm$ 1.1 | -22 $\pm$ 19% | 4 $\pm$ 103% | -59 $\pm$ 51% |
| **E-Beam sterilization** | | | | | | | |
| 1,12 % | 81.3 $\pm$ 0,2 | -3.64 | 267 $\pm$ 1.9 | 172 $\pm$ 1.3 | See above (data above tested relative to e-beam reference) | | |
| 1,00 % | 81.3 $\pm$ 0.2 | -4.08 | 264 $\pm$ 2.8 | 170 $\pm$ 1.9 | | | |
| 0,70 % | 76.1 $\pm$ 0.3 | -5.60 | 265 $\pm$ 1.9 | 176 $\pm$ 1.2 | | | |
| 0,50 % | 70.9 $\pm$ 1.2 | -9.60 | 265 $\pm$ 7.0 | 174 $\pm$ 3.5 | | | |
| 0,00 % | 45.7 $\pm$ 1.0 | -14.20 | 265 $\pm$ 3.5 | 174 $\pm$ 2.1 | | | |

**Contact angle $\theta$**

The contact angle of a hollow fiber membrane is determined by the capillary method, wherein the hollow fiber membrane serves as the capillary. The hollow fiber membrane is fixed in a measuring stand. Deionized water, stained with 0.25 mg/ml methylene blue, is filled into the trough disposed at the base of the measuring stand. The hollow fiber membrane, previously given a new cut edge transverse to the longitudinal extension by a straight razor, is immersed in the solution and the capillary height h determined after a 20-minute waiting period by ascertaining the height of the stained solution in the hollow fiber membrane above the liquid level of the test liquid in the trough. A new hollow fiber membrane is used after each measurement. The internal radius r of each hollow fiber membrane is determined at the cut edge by light microscopy.

The Young-Laplace equation for capillary pressure can be used to calculate the contact angle:

$$\rho gh = (2\gamma\cos\theta)/r$$

The equation for determining the contact angle from a given internal radius, capillary height and known constants can be defined as follows:

$$Arcos(\rho ghr/2\gamma)= \theta$$

wherein

p = water density at 25°C: 0.997 kg/m$^3$
g = gravitational acceleration 9.8 m/s$^2$
h = capillary height, m
$\gamma$ = surface tension of water at room temperature, 0.0728 N/m
r = capillary radius
$\theta$ = the contact angle to be determined.

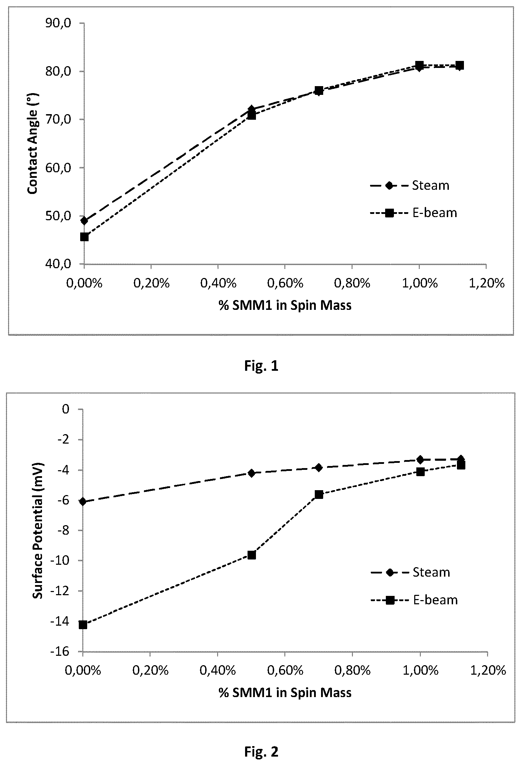

As shown in **Fig. 1,** the contact angle increased with increasing amounts of SMM1. The differences in contact angle of e-beam and steam sterilized dialyzers are only minor and within the statistical spread. The contact angles of the different samples increase with the increase of the amount of SMM1 within the spinning mass reaching a plateau the concentration range of 1 % w/w SMM1 and more. It has to be pointed out that in contrast to many publications for dialysis membranes it is desired to achieve a high contact angle when adding the fluorine- containing surface modifying molecules.

## Surface Potential (Zeta potential $\zeta$)

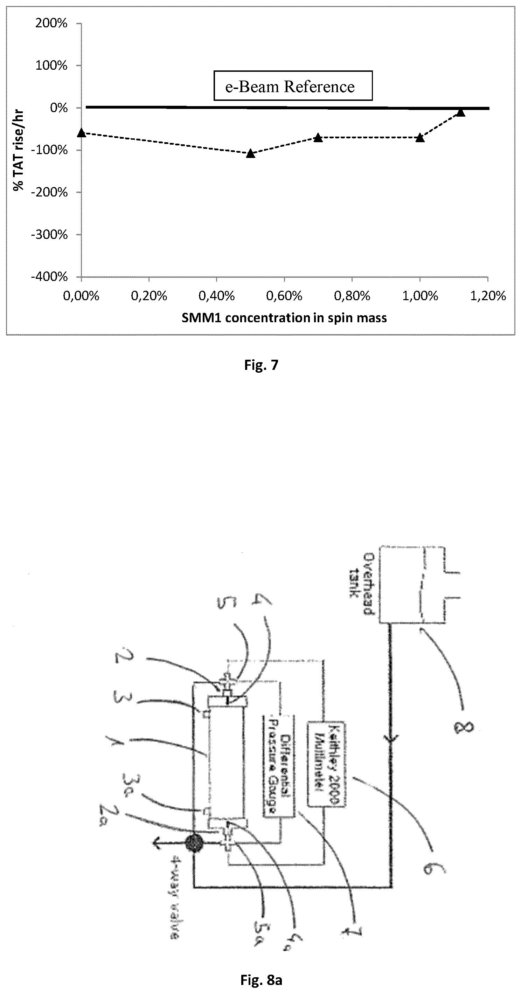

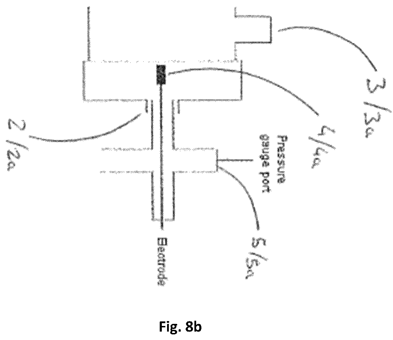

To determine the zeta potential of the hollow fiber membranes to be analyzed, a hollow fiber membrane filter (dialyzer) having 10,752 hollow fiber membranes with an internal diameter of 185 $\mu$m and a wall thickness of 35 $\mu$m is used. The hollow fiber membrane length relevant to measuring the zeta potential is 258 mm. The hollow fiber membranes are sealed at the ends in the hollow fiber membrane filter so as to create a first chamber encompassing the interior of the hollow fiber membranes and a second chamber encompassing the space between the hollow fiber membranes. Polyurethane from the Elastogran company (polyol C6947 and isocyanate 136-20) is used as the casting material. The casting height at each bundle end amounts to 22 mm. An apparatus in accordance with **Fig. 8a and b** is used for the measurement. The hollow fiber membrane filter 1 comprises fluid ports 2, 2a, 3, 3a to the respective first and second chamber of the hollow fiber membrane filter 1. The fluid ports to the first chamber of the hollow fiber membrane filter 1 are each provided with an Ag/AgCl electrode 4, 4a and a port for the pressure gauge 5, 5a as per **Fig. 2a.** The fluid ports 3, 3a to the second chamber of the hollow fiber membrane filter 1 are tightly sealed so that the second chamber of the hollow fiber membrane filter remains unfilled. The potential difference $\Delta E_z$ (mV) is thus registered between the two electrodes by a voltmeter 6, the decrease in pressure $\Delta P$ (N/m$^2$) between the access ways for the pressure gauge 5, 5a being registered by a manometer 7. The test liquid consists of a 1 mmolar KCl solution in water at a pH value of 7.4 and is provided in a tank 8 positioned approximately 1,000 mm above the filter. The pH value is set pursuant to the following rule: 50 mg $K_2CO_3$ is added to 100 liters of the KCl solution. The mixture is stirred in an open container until reaching a pH value of 7.4. The container is then tightly sealed. The measurement is performed at a temperature of 23°C +/- 2°C.

To measure the zeta potential, the test liquid is poured through a first fluid port 2 into the first chamber of the hollow fiber membrane filter which encompasses the interior space of the hollow fiber membranes and is routed out of the dialyzer again through a second fluid port 2a on the hollow fiber membrane filter connected to the interior space of the hollow fiber membranes. The hollow fiber membrane filter is initially flushed with the test liquid in this configuration for 10 min. until a stable value is reached, and if needed for an additional 5 min. The pressure difference and the potential difference are at the same time read from the manometer/multimeter and the zeta potential calculated therefrom. To increase measurement accuracy, it is provided to switch the two 4-way valves subsequent the measured value acquisition so as to effect a reverse flow of the test liquid through the interior space of the hollow fiber membranes. The measured value for the zeta potential is then formed from the mean measurement value in both flow directions. The zeta potential calculation is derived from the following equation:

$$\zeta = \frac{\eta * \Lambda o * d\ Ez}{\varepsilon o * \varepsilon r * d\ \Delta P}$$

where

$\zeta$ = zeta potential (mV)
$\eta$ = solution viscosity (0.001 Ns/m$^2$ )

$\Lambda_o$ = solution conductivity (A/(V*m))
$\varepsilon_o$ = vacuum permittivity ($8.85 * 10^{-12}$ A * s /(V * m))
$\varepsilon_r$ = relative solution permittivity ( 80 )
$E_Z$ = flow potential ( mV)
$\Delta_P$ = pressure difference ( $N/m^2$).

As shown in **Fig. 2,** the surface potential of the test dialyzers increased (became less negative), with the addition of more SMM1 to the spinning mass. E-beam sterilized dialyzers were considerably more negative without the addition of SMM1 (-14.20 mV at 0% SMM1 compared to -6.09 mV for steam sterilized dialyzers). It was surprisingly found that the surface potential reached a good level of almost -4 mV for the low concentration of 0.5% w/w. in the spinning mass, when the membranes where steam-sterilized. As of a concentration of 0.7% w/w. the surface potential was found to be higher than -4 mV. This value was reached only at a concentration of 1.12% w/w. within the spinning mass, when an e-beam sterilization method was performed. So it can be seen, that a desired surface potential can be achieved at a lower concentration of the SMM1 additive. Therefore a more cost effective dialyzer with high anti-thrombogenic properties is achieved.

## Clearance of Na and vitamin B12

The clearance of a hollow fiber membrane is determined on the basis of a hollow fiber membrane filter structured as per measurement method 2 according to DIN EN ISO 8637:2014. A sample dialyzer is prepared in the same way as for the determination of the zeta potential. Pursuant to 5.6.1.2 of the standard, aqueous sodium solutions at a concentration of 5 g/l NaCl and 0.05 g/l Vit B12 Vitamin B12 are used as test solutions for the blood area (blood area corresponds to the first chamber of the hollow fiber membrane filter encompassing the interior of the hollow fiber membranes); distilled water is hereby used for the dialysis fluid area (dialysis fluid area corresponds to the second chamber of the hollow fiber membrane filter encompassing the fiber interspace). The sodium concentration is determined by measuring conductivity. The vitamin B12 concentration is determined photometrically. The clearance tests make use of an identically structured hollow fiber membrane filter as is also used in the measurement pursuant measurement method 2. A flow of 300 ml/min is set in the first chamber of the hollow fiber membrane filter encompassing the interior of the hollow fiber membranes for the hollow fiber membrane filter produced within the scope of the present application, a flow of 500 ml/min is set in the second chamber of the hollow fiber membrane filter. Three measurements are performed for each embodiment.

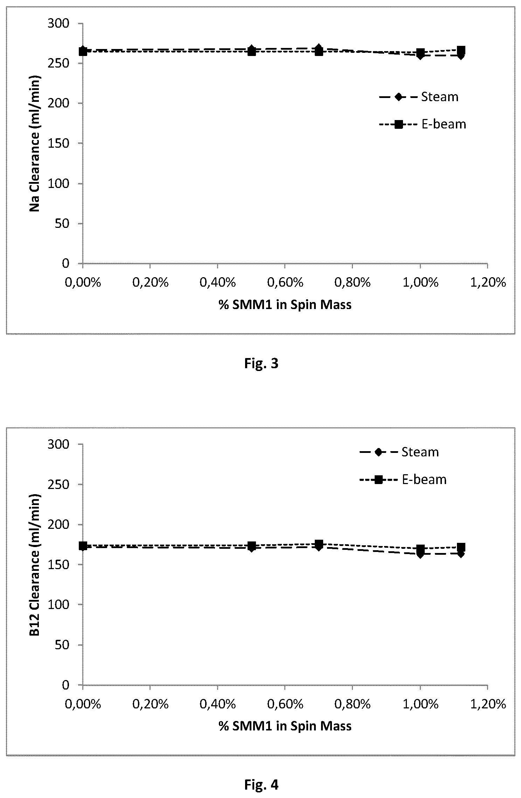

As shown in **Fig. 3** and **4,** all differences between the two sterilization methods are with the statistical spread, so that neither the addition of the surface-modifying molecule nor the method of sterilization influences the clearance of important ions and molecules. High flux dialyzers can be achieved by the inventive method.

## Hemocompatibility: Platelet loss, complement activation and TAT III

To determine the platelet loss and the complement activation, 450 ml of whole human blood is drawn using a 17G (1.5 mm) needle from healthy donors not taking any medication which could effect the coagulation of blood or the platelet properties. 750 IU heparin, diluted in 50 ml physiological saline solution, is provided in the blood collection bag such that a heparin concentration of 1.5 IU per ml results for the blood/saline solution mixture. The method for determining platelet loss is started within 30 min of the blood donation.

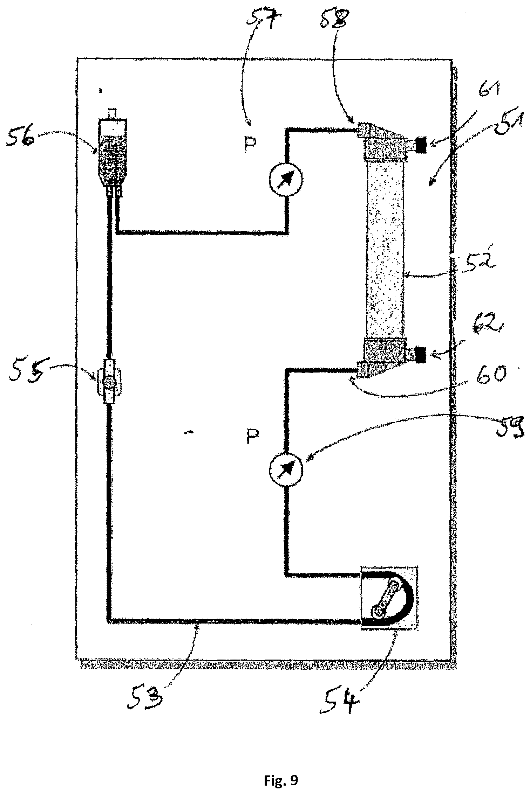

An apparatus 51 for determining platelet loss is constructed for the hollow fiber membranes to be tested as per the schematic depiction of **Fig. 9.** The apparatus comprises a dialyzer 52 with the hollow fiber membranes to be analyzed accommodated therein. The apparatus further comprises a system of hoses 53, a hose pump 54, a blood sample collection point 55, a reservoir for blood 56, a pressure sensor 57 at the blood inlet 58 of the dialyzer 52 and a pressure sensor 59 at the blood outlet 60 of the dialyzer 52. 200 ml of blood, which has first been heparinized as described above, is used in the determination. The blood is transported by the system of hoses 53 (material: PVC, manufacturer: Fresenius Medical Care, Germany) through the dialyzer 52 of the apparatus 51 by means of the hose pump 54 (manufacturer: Fresenius Medical Care, Germany). A new system of hoses is used for each measurement. The entire apparatus 51 is flushed for 30 min. prior to the measurement with a 0.9% (w/v) saline solution. To fill the apparatus with blood, the rinsing solution is drained and replaced with blood introduced into the apparatus at low pump speed until only pure blood fills the apparatus. The blood fill capacity is 200 ml. The displaced solution is discarded.

To prevent ultrafiltration during the analysis, the dialysate side is first filled with a 0.9% saline solution via ports 61 and 62 on the dialyzer and then sealed. The determination of the platelet loss ensues at 37°C, e.g. in an incubator (made by the Memmert company, Germany), over a period of 180 min., whereby samples are taken at the blood sample collection point 55 at the beginning of the measurement and then at 30, 60, 120 and 180 min. thereafter. The pressure at the blood inlet 58 and the blood outlet 59 is measured in order to ensure the conditions remain constant over the course of conducting the measurement. Should there be significant pressure changes, the reading must be rejected. The blood is pumped through the apparatus at a volumetric rate of 200 ml/min.

The hemocompatibility is determined using the complement activation C5a parameter, the platelet loss and TAT III determination. The platelet loss is determined by triple determination using an automatic hematology analyzer (K4500 Sysmex, Norderstedt, Germany).

The complement activation is determined by double measurement using an ELISA test kit (EIA-3327) from the DRG Instruments company, Marburg, Germany. The C5a factor, resulting from proteolytic C5 factor activation, serves as the measuring parameter.

The TAT III concentration is determined by double measurement using an ELISA test kit (Enzygnost TAT micro) from Siemens, Marburg, Germany.

During measuring, a further filter (either a FX60 from the Fresenius Medical Care company, Bad Homburg, Germany or a corresponding filter with the same concentration of SMM1, but with the reference sterilization method) is also measured as a reference using the second half of the blood donation and the measurement results determined (in %) relative to this reference filter. So doing enables mathematically compensating for the inherent wide fluctuations in blood reactions from different donors. Examples and comparative examples were produced using the same raw material batches.

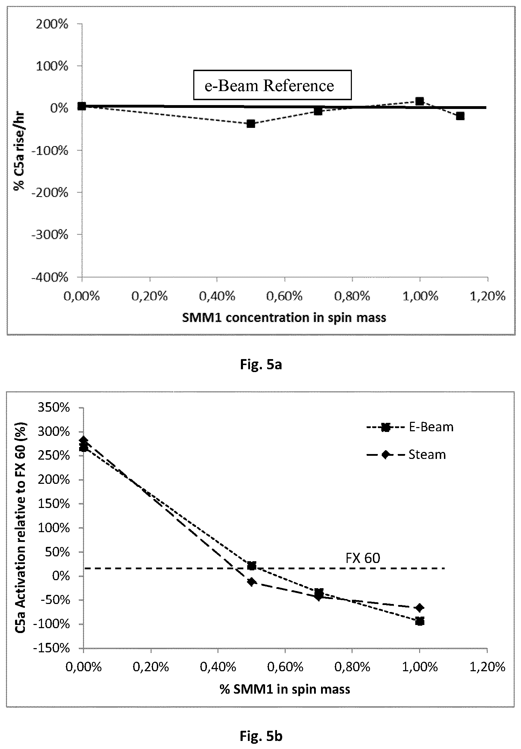

As shown in **Fig. 5a,** no noteworthy differences between the complement activation of the two dialyzers were observed as measured according to standard methods. For the determination of the complement activation for the sample filters, all samples were measured relative to a FX60 filter (Fresenius Medical Care, Bad Homburg, Germany). **Fig. 5a** does not reveal significant differences between the sterilization method "steam-sterilization" and "e-beam". The experiment teaches that both sterilization methods are well suited for the treatment of dialyzers with a good hemocompatibility. At a concentration of the SMM1 of 0.5% w/w. the same low complement activation is achieved as for the comparative FX60 filter, for higher concentrations a significant reduction of the complement activation can be seen compared to the FX 60 filter **(Fig. 5b).**

As shown in **Fig. 6a and b,** it could be surprisingly found in contrast to the parameter "complement activation", there are considerable advantages of steam sterilization over e-beam sterilization for the platelet loss. As shown in **Fig. 6b,** at a concentration of 0.5 % SMM1 *w/w* within the spinning mass the platelet loss is already greatly reduced for the steam-sterilized filter compared to the FX60 filter on the one hand and to the e-beam sterilized filter on the other hand. The e-beam sterilization exhibits still a higher platelet loss than the comparative FX60 filter. The difference between the sterilization methods are even increased when a concentration of the SMM1 within the spinning mass of 0.7 % is applied. Here the platelet loss is more than two times lower than for the FX60 filter and for the e-beam sterilized filter. As it is shown in **Fig. 6a,** the spinning mass has to comprise a SMM1 concentration of 1.12 % in order to achieve the same good platelet loss values as for the steam sterilized filter. This concentration is very high and within the solubility limit of the SMM1 molecule in the spinning mass according to the examples. An e-beam sterilized dialysis filter is therefore expensive to produce and is limited in production as far as the solubility of the surface modifying molecule is concerned.

As shown in **Fig. 7,** TAT III activation levels of steam sterilized dialyzers were lower compared to the e-beam sterilized filters. This improved anti-thrombogenic properties is shown almost over the range of different concentrations tested during the experiments. If an e-beam sterilization method is chosen for the production of a dialyzer, a concentration of the SMM1 molecule within the spinning mass of at least 1.12% has to be chosen to achieve the same good properties as for a steam-sterilized filter.

## Claims

1.   Method for manufacturing a dialysis filter comprising fluorine-containing hollow-fiber membranes, the fluorine-containing hollow-fiber membranes being made by a method comprising at least steps (A) to (C):

(A) preparing a spinning solution comprising an aprotic solvent, a hydrophobic base polymer, a hydrophilic polymer, and a fluorine-comprising surface modifying macromolecule in a concentration from 0.1 to less than 1.12 % w/w, based on the total weight of the spinning solution;
(B) extruding said spinning solution from an outer annular orifice through a tube-in-orifice spinneret into an aqueous solution, and
(C) isolating the formed hollow fiber membrane,

**characterized in that** the manufacturing of the dialysis filter comprises a steam sterilization procedure, and wherein the fluorine-containing spinning solution used in step (A) comprises a polysulfone. wherein the spinning solution used in step (A) comprises a polyvinylpyrrolidone, and

$$F_T\text{-}[B\text{-}(oligo)]_n\text{-}B\text{-}F_T$$

wherein

B comprises a urethane;
oligo comprises polypropylene oxide, polyethylene oxide or polytetramethylene oxide;
$F_T$ is a polyfluoroorgano group; and
n is an integer from 1 to 10.

2. The method of claim 1, wherein in step (A) said aprotic solvent is selected from the group consisting of dimethylformamide, dimethylsulfoxide, dimethylacetamide, N-methylpyrrolidone, or a mixture of two or more thereof.

3. The method of claim 1, where $F_T$ is represented by 1H,1H,2H,2H-perfluorooctanol, B is represented by a hexamethylene diisocyanate based urethane and oligo is represented by polypropylene oxide.

4. The Method of any one of the preceding claims, wherein after the method of making the hollow fiber membranes comprising a fluorine comprising surface modifying polymer according to at least steps (A) to (C) at least one of: contact angle $\theta$ of water on the surface of the hollow fiber membrane, the zeta potential $\zeta$, the platelet loss and/or the TAT III concentration is determined.

5. The method of any one of the preceding claims, where during the steam sterilization procedure a trans-membrane pressure difference is applied across the membrane walls of the hollow fiber membranes, such that water vapor is conducted into the interior of the hollow fiber membranes and is permeated through the membrane wall to the exterior of the fibers.

6. Dialysis filter comprising fluorine-containing hollow-fiber membranes comprising a hydrophobic base polymer, a hydrophilic polymer and a fluorine-comprising surface modifying macromolecule in a concentration of from 1.5 to less than 3.6 % w/w, based on the total weight of the fluorine-containing hollow fiber membrane

wherein the hollow fiber membrane is sterilized in a steam sterilization procedure, and
wherein the hydrophobic base polymer of the fluorine-containing hollow fiber membranes comprise a polysulfone polymer, and
wherein the hydrophilic polymer of the fluorine-containing hollow fiber membranes comprise a polyvinylpyrrolidone, and
wherein the surface-modifying macromolecule of the fluorine-containing hollow fiber membrane is described by formula

$$F_T\text{-}[B\text{-}(oligo)]_n\text{-}B\text{-}F_T$$

wherein

B comprises a urethane;
oligo comprises polypropylene oxide, polyethylene oxide or polytetramethylene oxide;
$F_T$ is a polyfluoroorgano group; and
n is an integer from 1 to 10.

7. Dialysis filter according to claim 6 wherein

$F_T$ is represented by 1H, 1H,2H,2H-perfluorooctanol,
B is represented by a hexamethylene diisocyanate based urethane and oligo is represented by polypropylene oxide.

8. Dialysis filter according to claim 6 or 7, wherein the fluorine containing hollow fiber membranes are **characterized in that**,

(X) the contact angle $\theta$ of water on the surface of the fluorine containing hollow fiber membrane is more than 70° or
(Y) the zeta potential $\zeta$ of the fluorine containing hollow fiber membranes is in the range of more than -5mV, preferably more than -4mV, or
(Z) the contact angle $\theta$ of water on the surface of the fluorine containing hollow fiber membrane is more than 70°, and the zeta potential $\zeta$ of more than - 5,0 mV, preferably of more than -4 mV,

wherein the contact angle of the hollow fiber membrane is determined by the capillary method, wherein the hollow fiber membrane serves as the capillary as described in the description.

**Patentansprüche**

1. Verfahren zur Herstellung eines Dialysefilters mit fluorhaltigen Hohlfasermembranen, wobei die fluorhaltigen Hohlfasermembranen nach einem Verfahren hergestellt werden, das mindestens die Schritte (A) bis (C) umfasst:

(A) Herstellen einer Spinnlösung, die ein aprotisches Lösungsmittel, ein hydrophobes Basispolymer, ein hydrophiles Polymer und ein Fluor enthaltendes oberflächenmodifizierendes Makromolekül in einer Konzentration von 0,1 bis weniger als 1,12 Gew.-%, bezogen auf das Gesamtgewicht der Spinnlösung, umfasst,
(B) Extrudieren der Spinnlösung aus einer äußeren ringförmigen Öffnung einer Rohr-in-Rohr Spinndüse in eine wässrige Lösung, und
(C) Isolieren der gebildeten Hohlfasermembran,
**dadurch gekennzeichnet, dass** die Herstellung des Dialysefilters ein Dampfsterilisationsverfahren umfasst, wobei die in Schritt (A) verwendete fluorhaltige Spinnlösung ein Polysulfon umfasst, wobei die in Schritt (A) verwendete Spinnlösung ein Polyvinylpyrrolidon und

$$F_T\text{-}[B\text{-}(oligo)]_n\text{-}B\text{-}F_T$$

umfasst, wobei
B Urethan aufweist,
oligo Polypropylenoxid, Polyethylenoxid oder Polytetramethylenoxid umfasst,
FT eine Polyfluororgano-Gruppe ist und
n ist eine ganze Zahl von 1 bis 10 ist.

2. Verfahren nach Anspruch 1, wobei in Schritt (A) das aprotische Lösungsmittel aus der Gruppe ausgewählt wird, die aus Dimethylformamid, Dimethylsulfoxid, Dimethylacetamid, N-Methylpyrrolidon oder einem Gemisch aus zwei oder mehreren davon besteht.

3. Verfahren nach Anspruch 1, wobei $F_T$ durch 1H,1H,2H,2H-Perfluoroctanol, B durch ein Urethan auf Hexamethylendiisocyanatbasis und oligo durch Polypropylenoxid verkörpert ist.

4. Verfahren nach einem der vorhergehenden Ansprüche, wobei nach dem Verfahren zur Herstellung der Hohlfasermembranen, die ein fluorhaltiges oberflächenmodifizierendes Polymer umfassen, gemäß mindestens den Schritten (A) bis (C) mindestens einer der folgenden Werte bestimmt wird: der Kontaktwinkel $\theta$ von Wasser auf der Oberfläche der Hohlfasermembran, das Zetapotenzial $\zeta$, der Thrombozytenverlust und/oder die TAT III-Konzentration.

5. Verfahren nach einem der vorhergehenden Ansprüche, wobei während des Dampfsterilisationsverfahrens eine transmembrane Druckdifferenz über die Membranwände der Hohlfasermembranen angelegt wird, so dass Wasserdampf in das Innere der Hohlfasermembranen geleitet wird und durch die Membranwand zur Außenseite der Fasern permeiert.

6. Dialysefilter mit fluorhaltigen Hohlfasermembranen, die ein hydrophobes Basispolymer, ein hydrophiles Polymer und ein fluorhaltiges oberflächenmodifizierendes Makromolekül in einer Konzentration von 1,5 bis weniger als 3,6 Gew.-%, bezogen auf das Gesamtgewicht der fluorhaltigen Hohlfasermembran, enthalten

wobei die Hohlfasermembran in einem Dampfsterilisationsverfahren sterilisiert wird, und
wobei das hydrophobe Basispolymer der fluorhaltigen Hohlfasermembranen ein Polysulfonpolymer umfasst, und
wobei das hydrophile Polymer der fluorhaltigen Hohlfasermembranen ein Polyvinylpyrrolidon umfasst, und
wobei das oberflächenmodifizierende Makromolekül der fluorhaltigen Hohlfasermembran durch die folgende Formel beschrieben wird

$$F_T\text{-}[B\text{-}(oligo)]_n\text{-}B\text{-}F_T$$

wobei
B Urethan aufweist,
oligo Polypropylenoxid, Polyethylenoxid oder Polytetramethylenoxid umfasst,
FT eine Polyfluororgano-Gruppe ist und
n ist eine ganze Zahl von 1 bis 10 ist.

7. Dialysefilter nach Anspruch 6, wobei $F_T$ wird durch 1H,1H,2H,2H-Perfluorooctanol, B wird durch ein Urethan auf Hexamethylendiisocyanatbasis und oligo durch Polypropylenoxid verkörpert ist.

8. Dialysefilter nach Anspruch 6 oder 7, wobei die fluorhaltigen Hohlfasermembranen **dadurch gekennzeichnet sind, dass**

(X) der Kontaktwinkel $\theta$ von Wasser auf der Oberfläche der fluorhaltigen Hohlfasermembran mehr als 70° beträgt, oder
(Y) das Zeta-Potential $\zeta$ der fluorhaltigen Hohlfasermembranen im Bereich von mehr als -5mV, vorzugsweise mehr als -4mV, liegt, oder
(Z) der Kontaktwinkel $\theta$ von Wasser auf der Oberfläche der fluorhaltigen Hohlfasermembran mehr als 70° beträgt und das Zetapotenzial $\zeta$ mehr als - 5,0 mV, vorzugsweise mehr als -4 mV beträgt,

wobei der Kontaktwinkel der Hohlfasermembran durch das Kapillarverfahren bestimmt wird, wobei die Hohlfasermembran als Kapillare dient, wie in der Beschreibung beschrieben.

## Revendications

1. Procédé de fabrication d'un filtre de dialyse comprenant des membranes à fibres creuses contenant du fluor, les membranes à fibres creuses contenant du fluor étant fabriquées par un procédé comprenant au moins les étapes (A) à (C) :

(A) la préparation d'une solution de filage comprenant un solvant aprotique, un polymère de base hydrophobe, un polymère hydrophile et une macromolécule de modification de surface comprenant du fluor, à une concentration comprise entre 0,1 et moins de 1,12 % p/p, sur la base du poids total de la solution de filage ;
(B) l'extrusion de ladite solution de filage à partir d'un orifice annulaire externe à travers une filière de type à tube dans l'orifice dans une solution aqueuse, et
(C) l'isolement de la membrane à fibres creuses formée,
**caractérisé en ce que** la fabrication du filtre de dialyse comprend une procédure de stérilisation à la vapeur, et dans lequel la solution de filage contenant du fluor utilisée à l'étape (A) comprend un polysulfone,
dans lequel la solution de filage utilisée à l'étape (A) comprend une polyvinylpyrrolidone, et

$$F_T\text{-}[B\text{-}(oligo)]_n\text{-}B\text{-}F_T$$

dans lequel
B comprend un uréthane ;
oligo comprend l'oxyde de polypropylène, l'oxyde de polyéthylène ou l'oxyde de polytétraméthylène ;
Ft est un groupe polyfluoroorganique ; et
n est un nombre entier compris entre 1 et 10.

2. Procédé selon la revendication 1, dans lequel, dans l'étape (A), ledit solvant aprotique est choisi parmi le groupe constitué de diméthylformamide, de diméthylsulfoxyde, de diméthylacétamide, de N-méthylpyrrolidone, ou d'un mélange de deux ou plus de ceux-ci.

3. Procédé selon la revendication 1, dans lequel Ft est représenté par le 1H,1H,2H,2H-perfluorooctanol, B est représenté par un uréthane à base de diisocyanate d'hexaméthylène et oligo est représenté par l'oxyde de polypropylène.

4. Procédé selon l'une quelconque des revendications précédentes, dans lequel, après le procédé de fabrication des membranes à fibres creuses comprenant un polymère de modification de surface comprenant du fluor selon au moins les étapes (A) à (C), au moins un élément est déterminé parmi : l'angle de contact θ de l'eau sur la surface de la membrane à fibres creuses, le potentiel zêta ζ, la perte de plaquettes et/ou la concentration en thrombine-antithrombine TAT III.

5. Procédé selon l'une quelconque des revendications précédentes, dans lequel, pendant la procédure de stérilisation à la vapeur, une différence de pression transmembranaire est appliquée à travers les parois de membrane des membranes à fibres creuses, de sorte que la vapeur d'eau est amenée à l'intérieur des membranes à fibres creuses et pénètre à travers la paroi de membrane vers l'extérieur des fibres.

6. Filtre de dialyse comprenant des membranes à fibres creuses contenant du fluor comprenant un polymère de base hydrophobe, un polymère hydrophile et une macromolécule de modification de surface comprenant du fluor, à une concentration comprise entre 1,5 et moins de 3,6 % p/p, sur la base du poids total de la membrane à fibres creuses contenant du fluor

dans lequel la membrane à fibres creuses est stérilisée au cours d'une procédure de stérilisation à la vapeur, et

dans lequel le polymère de base hydrophobe des membranes à fibres creuses contenant du fluor comprend un polymère de polysulfone, et

dans lequel le polymère hydrophile des membranes à fibres creuses contenant du fluor comprend une polyvinylpyrrolidone, et

dans lequel la macromolécule de modification de surface de la membrane à fibres creuses contenant du fluor est décrite par la formule

$$F_T\text{-}[B\text{-}(oligo)]_n\text{-}B\text{-}F_T$$

dans lequel

B comprend un uréthane ;
oligo comprend l'oxyde de polypropylène, l'oxyde de polyéthylène ou l'oxyde de polytétraméthylène ;
Ft est un groupe polyfluoroorganique ; et
n est un nombre entier compris entre 1 et 10.

7. Filtre de dialyse selon la revendication 6 dans lequel

Ft est représenté par le 1 H, 1H,2H,2H-perfluorooctanol,
B est représenté par un uréthane à base de diisocyanate d'hexaméthylène et oligo est représenté par l'oxyde de polypropylène.

8. Filtre de dialyse selon la revendication 6 ou 7, dans lequel les membranes à fibres creuses contenant du fluor sont **caractérisées en ce que**,

(X) l'angle de contact θ de l'eau sur la surface de la membrane à fibres creuses contenant du fluor est supérieur à 70° ou
(Y) le potentiel zêta ζ des membranes à fibres creuses contenant du fluor est dans la plage de plus de -5 mV, préférentiellement de plus de -4 mV, ou
(Z) l'angle de contact θ de l'eau sur la surface de la membrane à fibres creuses contenant du fluor est supérieur à 70°, et le potentiel zêta ζ est supérieur à -5,0 mV, préférentiellement supérieur à -4 mV,

dans lequel l'angle de contact de la membrane à fibres creuses est déterminé par la méthode capillaire, où la membrane à fibres creuses sert de capillaire tel que décrit dans la description.

**Fig. 1**

**Fig. 2**

**Fig. 3**

**Fig. 4**

Fig. 5a

Fig. 5b

**Fig. 6a**

**Fig. 6b**

**Fig. 7**

**Fig. 8a**

Fig. 8b

Fig. 9

Fig. 10

Fig. 11

Fig. 12

EP 3 911 432 B1

Fig. 13

32

**REFERENCES CITED IN THE DESCRIPTION**

**Patent documents cited in the description**

- EP 2295132 A1
- DE 102016224627

- DE 102016224627 A1

**Non-patent literature cited in the description**

- **KHULBE et al.** *J. Appl. Polym. Sci.*, vol. 104 (2), 710-721